# EUROPÄISCHE PATENTSCHRIFT

(11) **EP 0 878 187 B1**
(45) Veröffentlichungstag und Bekanntmachung des Hinweises auf die Patenterteilung: **27.06.2001**
(21) Anmeldenummer: 98106470.2
(22) Anmeldetag: 08.04.1998
(51) Int. Cl.: A61K 7/09

(54) **Verfahren zur dauerhaften Verformung von menschlichen Haaren**
Process for permanent waving of human hair
Procédé d'obtention de permanentes pour les cheveux humains

(30) Priorität: 19.04.1997 DE 19716497
(43) Veröffentlichungstag der Anmeldung: 18.11.1998
(73) Patentinhaber: GOLDWELL GmbH, 64297 Darmstadt (DE)
(72) Erfinder: Rose, Burkhard, 64297 Darmstadt (DE); Tennigkeit, J., Dr., 64342 Seeheim-Jugenheim (DE)

(56) Entgegenhaltungen:
- EP-A- 0 065 175
- FR-A- 2 718 351
- P. ALEXANDER: "permanent waving" MANUFACTURING CHEMIST, Bd. 59, Nr. 5, 1988, Seiten 61,63-64, XP002118531 London, GB

## Beschreibung

Die vorliegende Erfindung betrifft ein Verfahren zur dauerhaften Verformung von menschlichen Haaren, das sowohl eine gute Haarverformung als auch eine haarschonende Behandlung gewährleistet.

Die Dauerwellung erfolgt bekanntlich in zwei Behandlungsschritten, der reduktiven Spaltung der Cystin-Disulfidbrücken des Haares durch Einwirkung eines Reduktionsmittels, und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch die Thioglykolsäure, auch in Form ihrer Salze, insbesondere des Ammoniumsalzes, obwohl zahlreiche andere Thioverbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen oberhalb 7 und 10, insbesondere 8,5 bis 9,5, eingesetzt.

Da bei häufiger Anwendung von derartigen alkalischen Dauerwellmitteln Haarschädigungen auftreten können, besteht ein Bedarf nach Mitteln und Verfahren, die eine gute Dauerwellung bewirken, ohne das Haar zu schädigen.

FR-A-2 718 351 beschreibt ein Verfahren zur dauerhaften Verformung von Haaren, das bei häufiger Wiederholung sowohl eine gute Haarverformung als auch eine haarschonende Behandlung gewährleistet. Die Methode umfaßt das Auftragen einer sauren Zusammensetzung, die eine Carbonsäure als Fixiermittel enthält, auf das Haar.

Die vorliegende Erfindung geht von der Aufgabenstellung aus, ein Verfahren zum dauerhaften Verformen von menschlichen Haaren zu schaffen, das einerseits eine gute Verformungswirksamkeit aufweist, andererseits jedoch die Haarschädigung verringert und auch zur Entkräuselung, d.h. der Glättung, von Kraushaar verwendet werden kann.

Die Lösung dieser Aufgabe besteht darin, ein Verfahren zum dauerhaften Verformen von menschlichen Haaren durch Einwirkung eines Reduktionsmittels und anschließende Neutralisierung bzw. Fixierung durch Aufbringung eines Oxidationsmittels anzuwenden, das dadurch gekennzeichnet ist, daß auf das Haar zunächst eine wäßrige flüssige Zusammensetzung I, die mindestens ein Reduktionsmittel auf Basis einer organischen Thioverbindung enthält und einen alkalischen pH-Wert sowie eine niedere Viskosität, aufweist, auf das Haar aufgebracht, und nach etwa fünf- bis dreißigminütiger Einwirkungszeit auf das so vorbehandelte Haar eine weitere, viskose Zusammensetzung II, die ebenfalls mindestens ein Reduktionsmittel enthält und einen neutralen bzw. schwach sauren pH-Wert und eine höhere Viskosität als die Zusammensetzung I aufweist, aufgetragen wird.

Gemäß einer bevorzugten Ausführungsform liegt die Viskosität der Zusammensetzung I bei weniger als 50, insbesondere etwa 1 bis etwa 25 mPa.s, gemessen im Brookfield-Viskosimeter bei 20°C, sofern es sich um ein Dauerwellverfahren handelt. Handelt es sich dagegen um ein Verfahren zum Glätten von gekräuselten Haaren, kann die Viskosität der Zusammensetzung I auch etwas höher liegen, vorzugsweise zwischen etwa 1 und 1000, insbesondere 5 bis 500 mPa.s, gemessen im Brookfield-Viskosimeter bei 20°C.

Des weiteren weist die Zusammensetzung I vorzugsweise einen pH-Wert zwischen 7,1 oder 7.2 und 9.5, insbesondere 7.5 bis 9.0, auf.

Die Zusammensetzung II besitzt, sofern es sich um ein Dauerwellverfahren handelt, vorzugsweise eine Viskosität zwischen etwa 500 und etwa 15 000 mPa.s, insbesondere 1 000 bis 8 000, gemessen im Brookfield-Viskosimeter bei 20°C.

Handelt es sich hingegen um ein Verfahren zum Entkräuseln von Haaren, liegt die bevorzugte Viskosität bei etwa 5000 bis 150 000, insbesondere etwa 10 000 bis 125 000, vor allem etwa 15 000 bis 100 000 mPa.s, gemessen im Brookfield-Viskosimeter bei 20°C.

Der pH-Wert der Zusammensetzung [I liegt vorzugsweise zwischen 4,5 und 7, insbesondere 5 bis 5,5 und 7.

Aus der EP 0065 175 Al ist bereits ein Verfahren zur dauerhaften Verformung von Haaren bekannt, wobei das Haar vor oder nach dem Aufrollen auf Wickler mit einem flüssigen Haarkeratin reduzierenden Dauerverformungsmittel behandelt und anschließend auf die gewickelten Haare ein konsistentes Haarkeratin reduzierendes Dauerverformungsmittel mit einer Viskosität von 50 bis 5000 mPa.s bei 30°C und einer gegenüber dem flüssigen Dauerverformungsmittel stärkeren Verformungswirksamkeit aufgetragen wird.

Dieses Verfahren hat mit dem erfindungsgemäßen Verfahren ersichtlich keine näheren Berührungspunkte.

Durch das erfindungsgemäße Verfahren ist es insbesondere möglich, den zu erzielenden Reduktionsgrad besser zu kontrollieren, was vor allem bei strukturgeschädigten Haaren von Wichtigkeit ist.

Durch die Verwendung einer speziellen Kombinationsbehandlung mit alkalischem niederviskosen Dauerwellmittel und anschließendem Aufbringen einer neutralen oder sauren höherviskosen, insbesondere gel- oder emulsionsförmigen Zusammensetzung, kann eine Schädigung des Haares, wie sie bei Verfahren nach dem Stand der Technik, die ein alkalisches Wellmittel in der zweiten Stufe anwenden, beobachtet wird, nicht auftreten.

Die erfindungsgemäß eingesetzten Dauerwellmittel der Zusammensetzung I enthalten vorzugsweise eine organische reduzierende Thioverbindung. Bevorzugt sind Thioglykolsäure und Thiomilchsäure sowie deren Salze, insbesondere die Ammonium- und/oder Ethanolaminsalze.

Weitere einsetzbare Thioverbindungen sind insbesondere Cystein bzw. dessen Hydrochlorid, Homocystein, Cysteamin, N-Acetylcystein, Thioglycerin, Ethandiolmonothioglykolat, 1,2-Propylenglykolmonoglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Polyethylenglykol- wie Di-, Tri-und Tetraethylenglykolmonothioglykolate, Glycerinmonothiolactat und weitere Thiosäuren und deren Ester sowie Gemische derselben.

Gleiches gilt für die Reduktionsmittel in der Zusammensetzung II, in der auch die Verwendung anorganischer reduzierender Schwefelverbindungen wie Natriumhydrogensulfit prinzipiell möglich ist.

Der Gesamtgehalt an Reduktionsmitteln in den erfindungsgemäßen Zusammensetzungen beträgt üblicherweise 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel im Rahmen der Erfindung sind Ammoniumcarbamat, Ammoniak und/oder Ammonium(bi)carbonat.

Das erfindungsgemäße Verformungsverfahren ist sowohl zur Dauerwellung, also Lockung des menschlichen Haares, als auch zur Entkräuselung, also Glättung desselben, verwendbar, wobei bei letzterem, wie bereits ausgeführt, mit höher-viskosen Zusammensetzungen gearbeitet wird als beim Dauerwellverfahren.

Die Viskositätseinstellung erfolgt jeweils durch Zusatz entsprechender Mengen von an sich bekannten Verdickungsmitteln wie Cellulosederivaten, Polyelektrolyten, etc, oder vorzugsweise durch den Zusatz von C₁₀-C₂₂-Fettalkoholen, insbesondere im Gemisch mit langkettigen quaternären Ammoniumverbindungen, in einer bevorzugten Menge von 1 bis 5 Gew.-%, insbesondere 2 bis 4 Gew.-% Fettalkohol in Dauerwell- und 5 bis 15, insbesondere 7 bis 12 Gew.-% Fettalkohol in Entkräuselungsmitteln.

Die erfindungsgemäß zum Einsatz kommenden Dauerwellmittel enthalten vorzugsweise auch Tenside. Deren Anteil liegt bei etwa 0,1 bis etwa 10, insbesondere etwa 1 bis etwa 5 Gew.-% der das Reduktionsmittel enthaltenden Zusammensetzung.

Sowohl bei den in den Reduktionsmittel-Zusammensetzungen als auch bei den in den Fixiermitteln eingesetzten Tensiden handelt es sich vorzugsweise um die bekannten Produkte, die gegebenenfalls auch in Kombination untereinander zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete bevorzugte nichtionische Tenside, sind insbesondere C₈-C₁₈-Fettalkohol-Polyglykolether, Fettsäurepolyglykolester, Fettsäurealkanolamide, Aminoxide und vor allem C₈-C₁₈-Alkylpolyglucoside.

Es können auch amphotere Tenside wie die bekannten Betaine und Amidobetaine sowie, gemäß einer weiteren bevorzugten Ausführungsform, kationaktive Tenside wie quaternäre Ammoniumverbindungen, insbesondere in einer Menge von 0,05 bis 5, vor allem 0,1 bis 2,5 Gew.-%, berechnet auf die reduktionsmittelhaltige Zusammensetzung, eingesetzt werden.

Geeignete langkettige quaternäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumchlorid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Lauryltrimethylammoniumchlorid, Tri(oligooxyethyl)alkylammoniumphosphat, Cetylpyridiniumchlorid, etc.
Gut geeignet sind auch die in der EP-A 472 107 geoffenbarten quaternären Ammoniumsalze.

Im Prinzip sind alle quaternären Ammoniumverbindungen, wie sie im "CTFA International Cosmetic Ingredient Dictionary", 4th Ed. (1991), unter dem Trivialnamen "Quaternium" aufgeführt sind, geeignet.

Ein weiterer wünschenswerter Bestandteil der erfindungsgemäß verwendeten Reduktionsmittel-Zusammensetzungen sind, C₃-C₆-Alkandiole bzw. deren Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, 1,2-Pentandiol, Diethylenglykol und dessen Monomethyl- und Monoethylether sowie Dipropylenglykol und dessen Monomethyl- und Monoethylether.
Der Anteil dieser Diole liegt vorzugsweise zwischen 0,5 und 30, vorzugsweise etwa 1 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Mittels.

Die erfindungsgemäß eingesetzten Mittel können selbstverständlich alle in dauerhaften Verformungsmitteln üblichen Stoffe enthalten, auf deren detaillierte Aufzählung hier verzichtet wird, und liegen vorzugsweise als mehr oder weniger viskose Gele, Emulsionen bzw. Cremes vor.

Es kann sich dabei um einphasige Produkte oder um in getrennten Verpackungen untergebrachte Zusammensetzungen handeln, die bei der Anwendung vereinigt werden, wie sie z.B. in der DE-C 43 04 828 beschrieben sind.

Zur Vermeidung von Wiederholungen wird hierzu auf den Stand der Technik verwiesen, wie er beispielsweise in "Ullmanns's Encyclopedia of Industrial Chemistry", Vol. A12 (1986), S. 588 bis 591, sowie insbesondere in der Monographie von K. Schrader, "Grundlagen und Rezepturen der Kosmetika", 2. Auflage (1989, Hüthig-Verlag), S. 823 bis 840, sowie in dem Übersichtsartikel von D. Hollenberg et. al. in "Seifen-Öle-Fette-Wachse", 117 (1991), S. 81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzungen I und II aufgetragen. Nach deren Einwirkung und Spülung erfolgt die Fixierung mit üblichen, aus dem Stand der Technik hinreichend bekannten Peroxid- oder Bromat-Zusammensetzungen.

Ebenso kann selbstverständlich auch eine an sich bekannte Zwischenbehandlung zwischen Reduktions- und Neutralisierungsphase erfolgen.

Die folgenden Beispiele dienen der Illustration der Erfindung.

### Beispiel 1:

### Dauerwellverfahren für ungeschädigtes bis leicht poröses Naturhaar

Auf gewaschenes, auf Lockenwickler gedrehtes Haar wurde eine Dauerwell-Lösung der folgenden Zusammensetzung aufgebracht:

| | |
|---|---|
| Ammoniumthioglykolat | 9,0 (Gew.-%) |
| Ammoniumbicarbonat | 3,5 |
| PEG-35-Hydriertes Ricinusöl | 0,5 |
| Decylglucosid (P.G.: ∼1,5) | 1,0 |
| 1,2-Pentandiol | 1,5 |
| Polyquaternium-6 | 0,5 |
| Parfum | 0,5 |
| Ammoniak ad pH | 8,4 |
| Wasser | ad 100,0 |

Viskosität bei 20°C: <20 mPa.s (Brookfield-Viskosimeter Spindel Nr. 1; 20U/min.)
Nach zehnminütiger Einwirkung bei Raumtemperatur wurde, ohne vorheriges Spülen, ein neutrales Wellgel der folgenden Zusammensetzung aufgebracht:

| | |
|---|---|
| Ammoniumthioglykolat | 13,0 (Gew.-%) |
| Glycin | 2,0 |
| PEG-40-Hydriertes Ricinusöl | 0,5 |
| C₁₂-C₁₈-Fettalkohol | 3,5 |
| 1,2-Pentandiol | 1,5 |
| Polyquaternium-35 | 0,1 |
| Parfum | 0,4 |
| Monoethanolamin ad pH | 7,0 |
| Wasser | ad 100,0 |

Viskosität bei 20°C: 2200-3000 mPa.s (Brookfield-Viskosimeter, Spindel Nr. 5; 20U/min.)
Nach weiterer etwa zehnminütiger Einwirkung wurde gespült und mit einer handelsüblichen 2,5%-igen Wasserstoffperoxid-Lösung fixiert.

Nach dem Spülen und Trocknen wurde eine formschöne Dauerwellung erhalten.
Selbst bei fünffacher Wiederholung der Dauerwellung wurde, im Gegensatz zur Anwendung eines konventionellen alkalischen Dauerwellmittels, keinerlei Haarschädigung beobachtet.

### Beispiel 2

### Dauerwellverfahren für getönte, leicht geschädigte Naturhaare

| | |
|---|---|
| Ammoniumthioglykolat | 10,0 (Gew.-%) |
| Ammoniumthiolactat | 5,0 |
| Ammoniumhydrogencarbonat | 4,0 |
| 1,2-Propandiol | 1,5 |
| PEG-35-Hydriertes Ricinusöl | 0,5 |
| Isopropylalkohol | 3,5 |
| Polyquaternium-6 | 0,5 |
| Parfum | 0,5 |
| Ammoniak 25% ad pH | 7,7 |
| Wasser | ad 100,0 |

Viskosität bei 20°C: <25 mPa.s (Brookfield-Viskosimeter, Spindel Nr. 1; 20U/min.)

Diese Zusammensetzung wurde auf mit Lockenwicklern eingedrehtes Haar aufgebracht und nach etwa zehnminütiger Einwirkung, ohne vorheriges Ausspülen, mit einem Gel der folgenden Zusammensetzung behandelt:

| | |
|---|---|
| Ammoniumthiolactat | 16,0 (Gew.-) |
| Ammoniumchlorid | 1,0 |
| Glycin | 2,0 |
| C₁₂-C₁₆-Fettalkohol | 4,0 |
| Cocoamidobetain | 0,1 |
| PEG-40-Hydriertes Ricinusöl | 0,7 |
| 1,2-Pentandiol | 1,5 |
| Polyquaternium-7 | 0,2 |
| Parfum | 0,4 |
| Monoethanolamin ad pH | 6,5 |
| Wasser | ad 100,0 |

Viskosität bei 20°C: ca. 4000 mPa.s (Brookfield-Viskosimeter, Spindel Nr. 5; 20 U/min.)

Nach weiterer fünfminütiger Einwirkung bei Raumtemperatur wurde gespült, in üblicher Weise fixiert, gespült und getrocknet.

Es wurde eine formschöne Dauerwellung mit etwa den gleichen Eigenschaften wie diejenige nach Beispiel 1 erhalten.

### Beispiel 3:

### Entkräuselungs- und Haarglättungsverfahren

Auf gewaschenes Kraushaar wurde eine Zusammensetzung, bestehend aus

| | |
|---|---|
| Ammoniumthioglykolat | 9,0 (Gew.-%) |
| Ammoniumbicarbonat | 2,5 |
| Cocoamidobetain | 0,3 |
| Laureth-23 | 0,8 |
| Polyquaternium-2 | 0,4 |
| Parfum | 0,7 |
| Ammoniak (25%-ig) ad pH | 9,1 |
| Wasser | ad 100,0 |

Viskosität bei 20°C: <25 mPa.s (gemessen im Brookfield-Viskosimeter, Spindel Nr. 1, 5 U/min.) aufgebracht.

Nach fünfzehnminütiger Einwirkung wurde, ohne vorheriges Ausspülen, ein Gel der Zusammensetzung

| | |
|---|---|
| Thioglykolsäure | 8,0 (Gew.-%) |
| C₁₈-C₂₂-Fettalkohol | 12,0 |
| Oleth-50 | 2,5 |
| Laureth-23 | 1,0 |
| Stearyltrimoniumchlorid | 1,0 |
| 1,2-Pentandiol | 5,0 |
| Parfum | 0,7 |
| Monoethanolamin ad pH | 7,0 |
| Wasser | ad 100,0 |

Viskosität bei 20°C: 80 000 mPa.s (Brookfield-Viskosmieter, Spindel Nr.93C; 5U/min.) aufgebracht, weitere 10 Minuten permanent gekämmt, gespült und in üblicher Weise fixiert. Es wurde ein geglättetes Haar erhalten.

Bei fünfmaliger Wiederholung dieser Prozedur wurde keinerlei Haarschädigung beobachtet, während bei Durchführung des zweiten Verfahrensschritts mit einem alkalischen Glättungsmittel deutliche Haarschädigungen auftraten.

## Patentansprüche

1. Verfahren zum dauerhaften Verformen von menschlichen Haaren durch Einwirkung eines Reduktionsmittels und anschließende Neutralisierung bzw. Fixierung durch Aufbringung eines Oxidationsmittels, dadurch gekennzeichnet, daß auf das Haar zunächst eine niedrigviskose wäßrige flüssige Zusammensetzung I, die mindestens ein Reduktionsmittel auf Basis einer organischen Thioverbindung enthält und einen alkalischen pH-Wert aufweist, auf das Haar aufgebracht, und nach etwa fünf- bis dreißigminütiger Einwirkungszeit auf das so vorbehandelte Haar eine weitere, höherviskose Zusammensetzung II, die ebenfalls mindestens ein Reduktionsmittel enthält und einen neutralen bzw. schwach sauren pH-Wert und eine höhere Viskosität als die Zusammensetzung I aufweist, aufgetragen wird.

2. Verfahren zum Dauerwellen menschlicher Haare nach Anspruch 1, dadurch gekennzeichnet, daß die Zusammensetzung I eine Viskosität von weniger als 50mPa.s, gemessen im Brookfield-Viskositmeter bei 20°C, aufweist.

3. Verfahren zum Glätten gekräuselter menschlicher Haare nach Anspruch 1, dadurch gekennzeichnet, daß die Zusammensetzung I eine Viskosität von 1 bis 1 000 mPa.s, gemessen im Brookfield-Viskosimeter bei 20°C, aufweist.

4. Verfahren nach einem oder mehreren der Ansprüche 1 bis 3, dadurch gekennzeichnet, daß die Zusammensetzung I einen pH-Wert zwischen 7.1 und 9.5 aufweist.

5. Verfahren zum Dauerwellen von menschlichen Haaren nach einem oder mehreren der Ansprüche 1,2 und 4, dadurch gekennzeichnet, daß die Zusammensetzung II eine Viskosität zwischen etwa 500 und 15 000 mPa.s, gemessen im Brookfield-Viskosimeter bei 20°C, aufweist.

6. Verfahren zum Glätten gekräuselter Haare nach einem oder mehreren der Ansprüche 1,3 und 4, dadurch gekennzeichnet, daß die Zusammensetzung II eine Viskosität vzwischen etwa 5 000 bis 150 000 mPa.s, gemessen im Brookfield-Viskosimeter bei 20°C, aufweist.

7. Verfahren nach Anspruch 6, dadurch gekennzeichnet, daß die Zusammensetzung II eine Viskosität zwischen etwa 10 000 bis 100 000 mPa.s, gemessen im Brookfield-Viskosimeter bei 20°C, aufweist.

8. Verfahren nach einem oder mehreren der Ansprüche 1 bis 7, dadurch gekennzeichnet, daß die Zusammensetzung II einen pH-Wert zwischen 5 und 7 aufweist.

## Claims

1. Process for the permanent waving of human hair by effect of a reducing agent and subsequent neutralization or fixation, resp., by application of an oxidizing agent, wherein the hair is first treated with a low-viscosity, aqueous liquid Composition I containing at least one reducing agent on the basis of an organic thio compound and having an alkaline pH-value, and after about five to thirty minutes processing, the hair is treated with a higher viscosity Composition II, also containing at least one reducing agent and having a neutral or slightly acidic pH-value, and having a higher viscosity than Composition I.

2. Process for the permanent waving of human hair according to claim 1, characterized in that Composition I has a viscosity of less than 50 mPa.s, measured in the Brookfield viscosimeter at 20°C.

3. Process for the straightening of tightly curled human hair according to claim 1, characterized in that Composition I has a viscosity of 1 to 1,000 mPa.s, measured in the Brookfield viscosimeter at 20°C.

4. Process according to one or more of claims 1 to 3, characterized in that Composition I has a pH-value between 7.1 and 9.5.

5. Process for the permanent waving of human hair according to one or more of the claims 1, 2 and 4, characterized in that Composition II has a viscosity between about 500 and 15,000 mPa.s, measured in the Brookfield viscosimeter at 20°C.

6. Process for the straightening of tightly curled hair according to one or more of the claims 1, 3 and 4, characterized in that Composition II has a viscosity between about 5,000 to 150,000 mPa.s, measured in the Brookfield viscosimeter at 20°C.

7. Process according to claim 6, characterized in that Composition II has a viscosity between about 10,000 to 100,000 mPa.s, measured in the Brookfield viscosimeter at 20°C.

8. Process according to one or more of claims 1 to 7, characterized in that Composition II has a pH-value between 5 and 7.

## Revendications

1. Procédé de mise en forme durable des cheveux humains, par l'action d'un agent réducteur, puis par neutralisation ou, selon les cas, fixation ultérieure par application d'un agent oxydant, caractérisé en ce que l'on applique d'abord sur les cheveux une composition liquide aqueuse faiblement visqueuse I qui contient au moins un agent réducteur à base d'un composé thio organique et qui présente un pH alcalin, et en ce que l'on applique ensuite sur les cheveux ainsi prétraités, après un temps d'action d'environ cinq à trente minutes, une autre composition fortement visqueuse II, qui contient également au moins un agent réducteur et qui présente un pH neutre ou, selon les cas, faiblement acide et une viscosité supérieure à celle de la composition I.

2. Procédé pour permanenter des cheveux humains selon la revendication 1, caractérisé en ce que la composition I présente une viscosité inférieure à 50 mPa.s, telle que mesurée dans un viscosimètre rotatif Brookfield à 20 °C.

3. Procédé pour lisser des cheveux humains frisés selon la revendication 1, caractérisé en ce que la composition I présente une viscosité comprise entre 1 et 1 000 mPa.s, telle que mesurée dans un viscosimètre rotatif Brookfield à 20 °C.

4. Procédé selon une ou plusieurs des revendications 1 à 3, caractérisé en ce que la composition I présente un pH compris entre 7,1 et 9,5.

5. Procédé pour permanenter des cheveux humains selon une ou plusieurs des revendications 1, 2 et 4, caractérisé en ce que la composition II présente une viscosité comprise entre environ 500 et 15 000 mPa.s, telle que mesurée dans un viscosimètre rotatif Brookfield à 20 °C.

6. Procédé pour lisser des cheveux humains frisés selon une ou plusieurs des revendications 1, 3 et 4, caractérisé en ce que la composition II présente une viscosité comprise entre environ 5 000 et 150 000 mPa.s, telle que mesurée dans un viscosimètre rotatif Brookfield à 20 °C.

7. Procédé selon la revendication 6, caractérisé en ce que la composition II présente une viscosité comprise entre environ 10 000 et 100 000 mPa.s, telle que mesurée dans un viscosimètre rotatif Brookfield à 20 °C.

8. Procédé selon une ou plusieurs des revendications 1 à 7, caractérisé en ce que la composition II présente un pH compris entre 5 et 7.
